# EUROPEAN PATENT SPECIFICATION

(11) **EP 1 857 120 B1**
(45) Date of publication and mention of the grant of the patent: **28.12.2011**
(21) Application number: 06711869.5
(22) Date of filing: 18.01.2006
(51) Int. Cl.: A61K 47/14, A61K 9/08, A61K 31/22, A61K 31/245, A61K 31/43, A61K 31/431, A61K 31/546, A61K 31/7036, A61K 47/44, A61P 15/00, A61K 9/00

(54) **Infusion for mastitis**
Infusion für Mastitis
Perfusion pour mastites

(30) Priority: 19.01.2005 JP 2005011646
(43) Date of publication of application: 21.11.2007
(73) Proprietor: Nippon Zenyaku Kogyo Co., Ltd., Asaka-machi, Koriyama-shi Fukushima 963-0196 (JP)
(72) Inventor: TSUKADA, Junko, Nippon Zenyaku Kogyo Co., Ltd., Koriyama-shi, Fukushima, 963-0196 (JP); OHASHI, Eiichi, Nippon Zenyaku Kogyo Co., Ltd., Koriyama-shi, Fukushima, 963-0196 (JP); MATSUNAGA, Tomio, Nippon Zenyaku Kogyo Co., Ltd., Koriyama-shi, Fukushima, 963-0196 (JP)
(74) Representative: Walcher, Armin
(86) International application number: PCT/JP2006/300600
(87) International publication number: WO 2006/077856

(56) References cited:
- WO-A-98/09520
- JP-A- 01 153 625
- JP-A- 01 305 035
- JP-A- 52 083 924
- JP-A- 55 059 108
- JP-A- 56 100 712
- JP-A- 56 104 812
- JP-B1- 40 021 999
- US-A- 4 034 099
- DATABASE WPI Week 199637 Thomson Scientific, London, GB; AN 1996-368102 XP002495545 & JP 08 175989 A (NISSAN GOSEI KOGYO KK) 9 July 1996 (1996-07-09) & JP 08 175989 A (NISSAN GOSEI KOGYO KK) 9 July 1996 (1996-07-09)

## Description

### Technical Field

The present invention relates to infusion for mastitis in which dissolubility of a main ingredient from base is enhanced to enhance pharmacologic action of the main ingredient.

### Background Art

Conventionally, mastitis often develops in domestic animals raised for milking such as dairy cows and goats. Mastitis is a disease in which mammas of the domestic animals are infected with staphylococcus (e.g., *Staphylococcus aureus*), colon bacillus (*Escherichia coli*) or other pathogenic bacteria causing inflammation or a disease related to such bacteria. Mastitis is a very serious disease which may cause a matter of life or death for milk manufacturers due to its high incidence, and resultant reduced milk production, deteriorated milk quality and increased culling of diseased animals from dairy herds.

In order to prevent mastitis from developing, milk manufacturers have kept mammillae sanitized through various ways such as wiping or cleaning or immersing them into antiseptic solution. However, even if mammillae have been kept sanitized in such ways, it is still difficult to prevent mastitis from developing.

Thus, an established therapy frequently used upon development of mastitis is administration by infusing infusion for mastitis into mammas of domestic animals affected with mastitis. Most of infusion for mastitis is a turbid mixture of a main ingredient comprising antibiotic with an oily base such as canola oil. By directly infusing such infusion for mastitis into mammas, most of mastitis is cured or alleviated.

In old times, used as a main ingredient of infusion for mastitis was penicillin antibiotic and then synthesized penicillin antibiotic prevailed; thereafter, it was replaced by cephem antibiotic. Nowadays, cephem antibiotic is most frequently used.

The oily base used is corn oil, canola oil, peanut oil, olive oil, cotton seed oil or the like.

In old times, used as an infusion barrel was an aluminum tube with a capped needle; then, used was an aluminum tube with a one-touch cap. Nowadays, a syringe is frequently used.

Prior art for infusion for mastitis has been disclosed, for example, in References 1 and 2.
[Reference 1] JP 2001-206849A
[Reference 2] JP 2001-511451A

US 4,034,099 describes compositions and methods for treating mastitis in milk animals which comprises administering by intramammary infusion an effective amount of an anti-mastitis medicament dispersed in a vehicle comprising an oil, a fatty acid ester and, optionally, a fatty acid salt.

JP 08-175989 describes a dipping liquid composition for preventing mammilitis comprising monoglyceride caprylate and/or monoglyceride caprate and monoglyceride laurate.

### Summary of the Invention

### Problems to be Solved by the Invention

Generally, most of the established infusion for mastitis is, as mentioned above, a mere turbid mixture of a main ingredient with an oily base; no substantial ingenuity has been made as to the base.

As a result, in the above-mentioned conventional infusions for mastitis, dissolubility (releasability) of a main ingredient from a base is low and thus absorbency of the main ingredient is also low, so that no sufficient pharmacologic action by the main ingredient can be expected. Such low dissolubility and absorbency disadvantageously result in substantial increase in consumed amount of the main ingredient and prolongation of its residence term in animal body.

The invention was made in view of the above and has its object to provide infusion for mastitis in which diffusivity or dispersivity of a base is improved to enhance dissolubility of a main ingredient, thereby enhancing pharmacologic action of the main ingredient.

### Means or Measures for Solving the Problems

The invention is directed to infusion for treating mastitis which comprises cephem antibiotic as a main ingredient, medium-chain fatty acid monoglyceride having carbon atoms in the range of C8 through C12 and an oily base.

It is preferable in the above means that an amount of the medium-chain fatty acid monoglyceride is 0.5% by weight or more of total.

### Effects of the Invention

According to infusion for mastitis of the invention, which comprises a main ingredient, medium-chain fatty acid monoglyceride and an oily base, diffusivity or dispersivity of the main ingredient is improved by the monoglyceride to enhance dissolubility and thus absorbency of the main ingredient. As a result, the infusion of the invention has excellent effects and advantages, in comparison with conventional infusions for mastitis, that it has immediate effectivity and is short in residual term and that the main ingredient can be expected to be utilized effectively and without waste, thereby reducing a consumed amount of the main ingredient.

### Brief Description of the Drawings

[Fig. 1] A diagram showing dissolution ratio of CEZ when MCM concentrations are varied stepwise from 0 to 10% by weight in dissolution test, Method 2 (puddle method).
[Fig. 2] A diagram showing transition of CEZ concentrations in blood of cows with respect to infusion for mastitis.
[Fig. 3] A diagram showing transition of CEZ concentrations in milk of cows with respect to infusion for mastitis.
[Fig. 4] A diagram showing a dissolution amount of CEZ in dissolution test, Method 2 (puddle method).
[Fig. 5] A diagram showing a dissolution ratio of CEZ in dissolution test, Method 2 (puddle method).
[Fig. 6] A diagram showing transition of CEZ concentrations in milk of cows in administrations to the cows with different CEZ ratios.
[Fig. 7] A diagram showing transition of CEZ concentrations in milk.
[Fig. 8] A diagram showing transition of CEZ concentrations in blood.
[Fig. 9] A diagram showing transition of Cmax in administrations.

### Best Mode for Carrying Out the Invention

The invention is directed to infusion for mastitis with a main ingredient dispersed to or dissolved in a mixture of oily base with medium-chain fatty acid monoglyceride, additive being added thereto as needs demand. The main ingredient of the infusion for mastitis includes cephem.

The cephem antibiotic usable may be conventionally used cefalonium, cefapirin benzathine, cefapirin sodium, cefazolin, cefuroxime sodium or the like.

The oily base serves for effective dispersion of the main ingredient and to add satisfactory flowability thereto as infusion for mastitis. The oily base usable may be conventionally used corn oil, canola oil, peanut oil, olive oil, cotton seed oil or the like.

The additive usable may be colorant, dispersant or the like.

The medium-chain fatty acid monoglyceride (hereinafter referred to as MCM) is a kind of glycerin fatty acid ester; to admix MCM with an oily base can be expected to bring about effect as diffusing agent for enhancing dissolubility (releasability) of the main ingredient. More specifically, MCM has been known to have effects such as interfacial diffusion, facilitation of absorption of hardly absorbable drugs and antibacterial activity and therefore has been used as percutaneous absorbent, suppository base or the like. However, there have been no production nor disclosure up to the present on infusion for mastitis with MCM as in the invention.

There are MCMs having carbon atoms in the range of C8 through C12 any of which may be used in the invention. MCM commercially available is, for example, Sunsoft 707 (product of Taiyo Kagaku Co., Ltd.) which is monoester of caprylic acid with 8 carbon atoms or Sunsoft 757 (product of Taiyo Kagaku Co., Ltd.) which is monoester of lauric acid with 12 carbon atoms. The invention is not limited to such MCMs and may employ any other various kinds of monoglyceride. MCM, which itself has weak emulsifiability, has function that mixing it in small amount to oil phase lowers surface tension of the phase; the oil phase with MCM has dispersive tendency at phase boundary due to lowered surface tension.

Thus, the inventors, who hoped that oily base with MCM may enhance dispersivity of the base and releasability of main ingredient, conducted various tests for validation.

As a result, it was surprisingly revealed that this can provide infusion for mastitis which not only has originally expected improvement in releasability of main ingredient in combination with enhanced facilitation of absorbency of main ingredient, but also has immediate effectivity and short residual term in animal body and can obtain satisfactory pharmacologic action with use of main ingredient in a smaller amount, thus completing the invention.

Examples of the invention will be disclosed hereinafter minutely. However, the invention is not limited to those examples.

### [Example 1]

Sunsoft 707 (product of Taiyo Kagaku Co., Ltd.) which is monoester of caprylic acid with 8 carbon atoms was used as MCM to prepare infusion 1 (3 g) with composition shown in Table 1.

**Table 1**

| infusion 1 | | |
|---|---|---|
| main ingredient: | cefazolin (CEZ) | 150 mg (potency) |
| MCM: | | 75 mg (2.5% by weight) |
| Additive: | food blue No. 1 | 25 mg |
| oily base: | canola oil | remainder |
| total: | | 3 g |

A plastic syringe of the type ordinary used for ointment for mastitis in the applicant company was charged with the above infusion 1 to prepare a prototype of infusion for mastitis. The prototypical infusion 1 exhibited physical properties suitable for use as infusion for mastitis.

### 1. Dissolubility test with various MCM ratios:

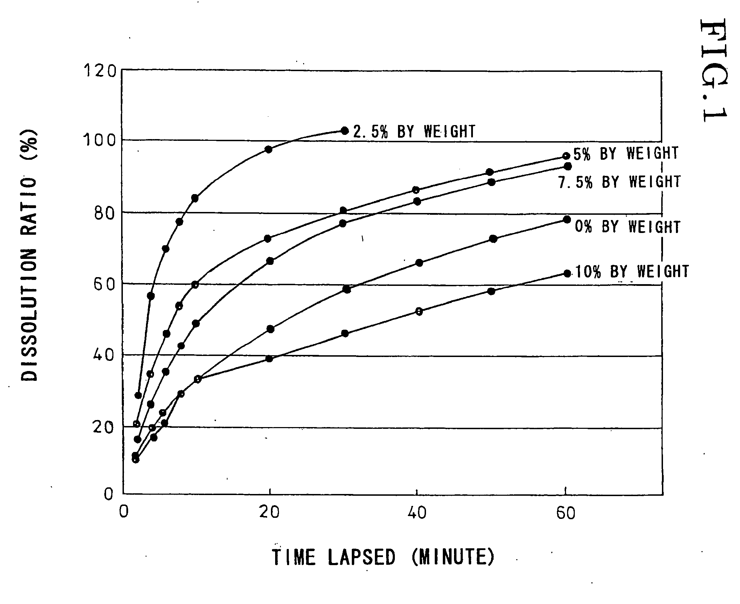

As to infusion 1, test was made on the basis of dissolution test, Method 2 (puddle method) of Japanese Pharmacopoeia general test methods. Test liquid used was phosphate buffer (pH 6.5) (a mixture of 4.86 g of potassium dihydrogenphoshate (product of Wako Pure Chemical Industries, Ltd.) with 5.12 g of disodium hydrogenphosphate 12-water (product of Wako Pure Chemical Industries, Ltd.) in 1 L of distilled water, pH being adjusted). The test was conducted with revolution speed of 25 r.p.m.; test liquid was sampled with time lapsed to carry out measurement of CEZ concentration dissolved in the test liquid, i.e., dissolution ratio of CEZ according to liquid chromatograph of potency examination in Animal Antibiotic Medical Supplies Standard (hereinafter referred to as HPLC method: high-performance liquid chromatography method). The test was conducted with variety of MCM concentrations in stepwise in a range of 0-10% by weight. The results are shown in Fig. 1.

In Fig. 1, prescription with highest dissolution ratio was that with 2.5% by weight of MCM. It was predicted in the test that prescription with 10% by weight of MCM would exhibit best dissolution since the higher the MCM concentration is, the higher the dispersivity of the main ingredient is; contrary to such prediction, prescription with 2.5% by weight of MCM exhibited the best dissolubility. This is considered to be owing to the fact that a feature as dissolution helper or solubilizer for hardly-soluble drugs, which is one of features of MCM, affects on dissolution of CEZ.

### 2. Test on transition of CEZ concentrations in blood and in milk with respect to administrations with MCM to cows:

As to infusion 1, test was conducted in the following manner on transition of concentrations in blood and in milk. Reagents used were products of Wako Pure Chemical Industries, Ltd. unless otherwise specified.

### · Measurement method (HPLC method) on transition of concentrations in blood:

In order to determine transition of concentrations in blood, blood serum analysis was conducted. The following solutions were prepared beforehand.

### 1) Preparation of sample solution:

Accurately measured 2 ml of blood serum, 87.1 g of potassium dihydrogenphosphate (product of Nacalai Tesque, Inc.), 39.9 g of citric acid monohydrate (product of Nacalai Tesque, Inc.) were dissolved in distilled water to make 1000 ml of solution in total. The solution was added and mixed with 20 ml of phosphate buffer, pH 5.3, having been adjusted to pH 5.3±0.1 by 1N KOH, and was introduced into a solid-phase extraction column, Sep-pak plus C18 cartridge (product of Waters Inc.) which has been treated beforehand with 5 ml of methanol and 10 ml of water. After the introduction, the column was washed with 20 ml of water and then CEZ was dissolved by 5 ml of acetonitrile and the solvent was evaporated under reduced pressure at a temperature of 40°C or less. To the residue, 7.0 g of potassium dihydrogenphosphate and 6.0 g of disodium hydrogenphosphate 12-hydrate were dissolved in distilled water to make 1000 ml of solution in total. The solution was added with accurately measured 1 ml of phosphate buffer, pH 6.0, which has been adjusted beforehand to pH 6.0±0.1 by phosphoric acid, re-dissolved by ultrasonic treatment (1 min.) and filtered by Ekicrodisc 13 CR (0.45 µm membrane filter; product of Nihon Pall Ltd.) to provide a sample solution.

### 2) Preparation of reference solution:

Precisely measured 25 mg (potency) of working reference cefazolin, 3.4 g of potassium dihydrogenphosphate and 10.65 g of disodium hydrogenphosphate (anhydrous) were dissolved in distilled water to make 1000 ml of solution in total. The solution was dissolved in phosphate buffer, pH7, having been adjusted to pH 6.0±0.1 by phosphoric acid to prepare a reference solution with definite concentration of 500 µg (potency)/ml. An appropriate amount of reference stock solution was precisely measured and was diluted stepwise by phosphate buffer, pH 6, to prepare 5.0, 1.0, 0.5, 0.25 and 0.10 µg (potency) /ml each of reference solution.

Test was conducted through liquid chromatography to 200 µl each of prepared sample and reference solutions under the following conditions to determine peak area of CEZ. By regression formula of analytical curve prepared from peak area of chromatogram of the reference solution, the CEZ concentration in the sample solution was calculated.
[HPLC conditions (ion-pair gradient)]
detection wavelength: UV 270 nm
column: Inertsil PH 5 µm 4.6 × 250 nm (product of GL Sciences Inc.)
column temperature: 25°C
flow rate: 1 ml/l min.
mobile phase:
Liquid A (pH 3.0, phosphoric acid)
TBA Liquid : acetonitrile = 9 : 1
Liquid B (pH 3.0, phosphoric acid)
TBA Liquid : acetonitrile : methanol - 55 : 30 : 15
TBA Liquid was a solution prepared by dissolving 0.34 g of tetrabutylammonium sulfate (product of Nacalai Tesque, Inc.) and 17.9 g of disodium hydrogenphosphate 12-hydrate in distilled water to provide 1000 ml of solution in total.

Time lapsed and change in concentration of Liquid B are as shown in Table 2.

**Table 2**

| time program (analysis time: 45 min.) | |
|---|---|
| time lapsed (min.) | concentration (%) of Liquid B |
| 12 | 8 |
| 25 | 20 |
| 30 | 20 |
| 35 | 8 |
| 44 | 8 |

### ·Measurement of transition in concentration in milk (bioassay method):

With respect to infusion 1, milk analysis was conducted so as to examine transition in concentration in milk. The following solutions were prepared beforehand.

### 1) Culture medium and buffer solution:

Culture medium for passage reservation: Mil-P slant agar culture medium (Mil-P No. 1 culture medium)(product of Kyokuto Pharmaceutical Industrial Co., Ltd.: this also applies to culture mediums mentioned hereinafter.)

### Growth culture medium: Mil-P strain broth culture medium (Mil-P No. 2 culture medium)

### Test culture medium: Mil-P disc agar culture medium (Mil-P No. 3 culture medium)

Phosphate buffer (pH 6.0): Dissolved in about 750 ml of distilled water were 3.5 g of potassium dihydrogenphosphate and 3.0 g of disodium hydrogenphosphate 12-water. The solution was adjusted to pH 6.0±0.1 by phosphoric acid and added with distilled water to prepare 1000 ml of solution.

### 2) Test strain:

### Test strain: Bacillus stearothermophilus var. calidolactis C-953

Preparation of test strain liquid: Test strain with repeated passage on reservation culture medium was propagated, upon usage, on the growth culture medium and cultured at 55±1°C for 17±1 hours into the strain liquid.

### 3) Preparation of flat test plates:

Test culture medium kept warm at about 55°C was mixed with test strain liquid at a ratio of 5 : 1. The mixture was dispensed each by 10 ml into sterilized plastic Petri dishes with inner diameter of 90 mm and cooled and solidified on horizontal plates. Then, each of the plates was formed with four holes with a diameter of 8 mm by using a drill (product of Toyo Sokuteiki Co., Ltd.) such that the holes were situated on respective apexes of a substantially cyclic square in a circle with a radius of about 25 mm about a center point of the flat plate, thereby providing the flat test plates.

### 4) Preparation of test solution:

Sample frozen at -15°C or less till just before measurement was allowed to melt at room temperature, and 1 ml of the sample was accurately measured in a stoppered sedimentation tube. This was added with 20 ml of methanol, was shaken for 10 minutes and centrifuged at 3000 r.p.m. for 5 minutes; and supernatant liquid was transferred to a pear-shaped flask. This was added with 1 ml of n-propanol (product of Kanto Chemical Co., Inc.) and evaporated under reduced pressure at a temperature of 45°C or less. The residue was added with and dissolved in 5 ml of phosphate buffer (pH 6.0) to provide sample solution.

### 5) Preparation of reference solution:

An appropriate amount of working reference cefazolin was dissolved in a small amount of acetone (product of Fisher Scientific International, Inc.), and further diluted with phosphate buffer (pH 6.0) to prepare about 1 mg (potency)/ml of reference stock solution with definite concentration. The stock solution was reserved at 5°C or less, and was used within 10 days after the preparation. Just before use, an appropriate amount of the reference stock solution was accurately measured and accurately diluted with phosphate buffer (pH 6.0) into dilute solutions each with 0.16, 0.08, 0.04, 0.02 and 0.01 µg (potency)/ml concentration to thereby provide reference solutions. Aside from this, an appropriate amount of reference stock solution was accurately measure and accurately diluted with phosphate buffer (pH 6.0) to prepare dilute solution with 0.04 µg (potency)/ml concentration as central working reference dilute solution.

The prepared solutions were used to conduct milk analysis through microbioassay method. More specifically, the holes drilled on the test flat plats were charged with 50 µl each of sample and reference solutions and cultured at 55±1°C for 55 hours or more. Diameters of developed inhibition zones were measured, and regression equation of calibration curve was determined from inhibition zone diameter of the reference solution. Inhibition zone diameter of the sample solution was substituted in the equation to calculate CEZ concentration in the sample solution. The calculated CEZ concentration was multiplied by dilution strength upon re-dissolution to obtain CEZ concentration in the sample. When definite inhibition zones were not obtained in the test results and when calculation results were less than measurable limit, the scores were recorded as ND (not detectable).

In the conditions shown in the following Table 3, infusion 1 (with 2.5% by weight of MCM and 150 mg of CEZ) and cefamezin QR as contrast drug (cefazolin oily infusion containing no MCM and 150 mg of CEZ: product of Nippon Zenyaku Kogyo Co., Ltd.) were infused into mammas of five Holstein dairy cows to examine transition of CEZ concentrations in blood and in milk.

**Table 3**

| Test animal | five Holstein dairy cows |
|---|---|
| Test drug | infusion 1: with 2.5% by weight of MCM and 150 mg of CEZ control drug: cefamezin QR (150 mg of CEZ) |
| Administration | administration through infusion to 4 mammas with each specimen drug (150 mg potency) per mamma |
| Sampling | blood: before administration and 0.5, 1, 2, 3, 4, 6, 8 and 24 hours after administration milk: before administration and 6, 24, 30. 48, 54 and 72 hours after administration |
| Analysis | blood serum: HPLC method milk: bioassay method |

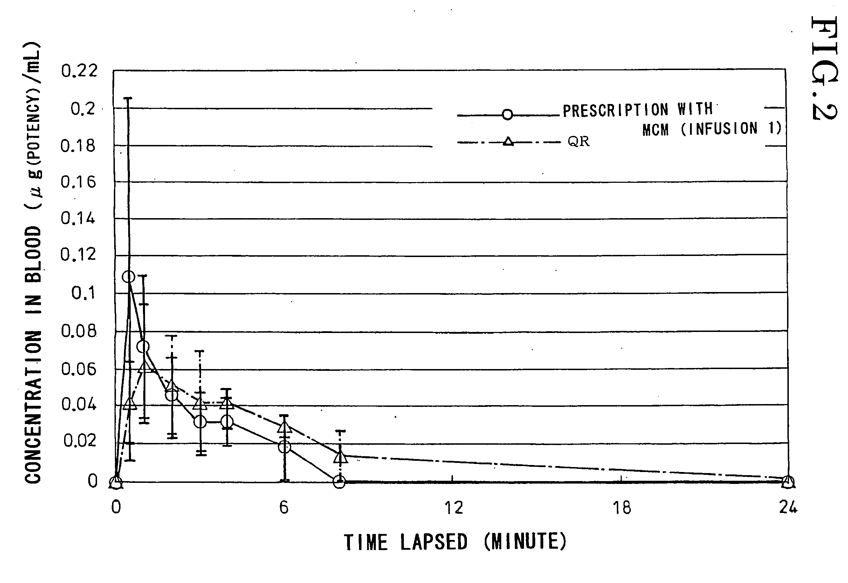

Fig. 2 shows results of transition of CEZ concentrations in blood of cows administered with infusion 1 for mastitis; detectable limit was 0.015 µg (potency)/ml and values higher than that were recorded as data.

It is revealed from the results in Fig. 2 that, in comparison with cefamezin QR, prescription with MCM has early start in rising of concentration in blood and its maximum concentration (Cmax) in blood is higher.

From this it is revealed that infusion 1 with 2.5% by weight of MCM can rapidly enhance CEZ concentration in blood in shorter time and thus main ingredient is expected to have a rapid-acting pharmacologic effect.

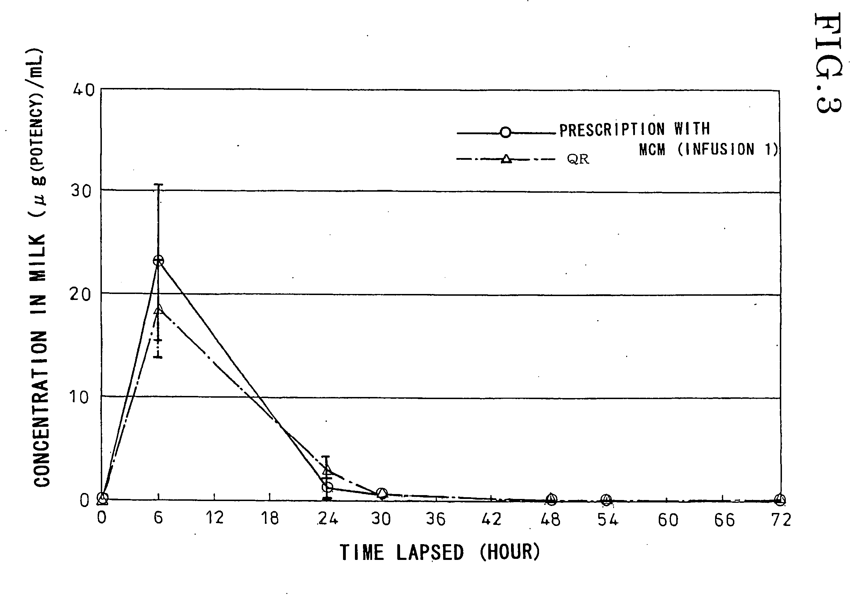

Fig. 3 shows results in transition of CEZ concentrations in milk of cows administered with infusion 1 for mastitis and cefamezin QR.

In Fig. 3, no substantial differences were seen between administration groups. Prescription with MCM has higher concentration in milk than that of cefamezin QR, which fact seems to reveal that the former has higher dissolution into milk than cefamezin QR. This is in conformity with results of dissolution test.

Infusion 1 with 2.5% by weight of MCM has rapid start in rising CEZ concentration in blood in comparison with current cefamezin QR and has significant difference in maximum concentrations in blood and in milk. It was revealed that CEZ, which is a bactericidal antibacterial agent, can exhibit pharmacologic effects, providing that sufficient concentration can be kept in blood and in milk for a short period of time; infusion 1 which can, as mentioned in the above, have rapid start in rising of concentration in blood and keep the maximum concentration in blood is much effective in treatment of mastitis. It was revealed that infusion 1 with 2.5% by weight of MCM can keep high utilization ratio of the main ingredient since it is superior in diffusivity or dispersivity and has main ingredient easily dissolubable into milk and has higher absorbency.

In view of the above, the inventors conjectured that infusion even with reduced amount of CEZ may exhibit effect similar to that of current cefamezin QR, and conducted the following tests.

Each 1 g of infusions, i.e., prescription (infusion 1) with CEZ content of 150 mg/3g same as that of conventional prescription, one half prescription (infusion 2) with CEZ content of 75 mg/3g as shown in Table 4 below and one third prescription (infusion 3) with CEZ content of 50 mg/3g as shown in Table 5 below and cefamezin QR as contrast drug, were separately charged and dissolution amount and dissolution ratio to test liquid were measured with predetermined time interval.

### [Example 2]

**Table 4**

| infusion 2 | | |
|---|---|---|
| main ingredient: | cefazolin (CEZ) | 75 mg (potency) |
| MCM: | | 75 mg (2.5% by weight) |
| additive: | food blue No. 1 | 25 mg |
| oily base: | canola oil | remainder |
| total: | | 3 g |

A plastic syringe for mastitis ointment ordinarily used in the applicant company was charged with the above infusion 2, thereby experimentally prepared infusion for mastitis. The experimentally prepared above-mentioned infusion 2 exhibits physical properties suitable for use as infusion for mastitis.

### [Example 3]

**Table 5**

| infusion 3 | | |
|---|---|---|
| main ingredient: | cefazolin (CEZ) | 50 mg (potency) |
| MCM: | | 75 mg (2.5% by weight) |
| Additive: | food blue No. 1 | 25 mg |
| oily base: | canola oil | remainder |
| total: | | 3 g |

A plastic syringe for mastitis ointment always used in the applicant company was charged with the above-mentioned infusion 3 to experimentaliy prepare infusion for mastitis. The above-mentioned experimentaliry prepared infusion 3 exhibits physical properties suitable to use as infusion for mastitis.

### 1. Dissolubility test with various MCM ratios:

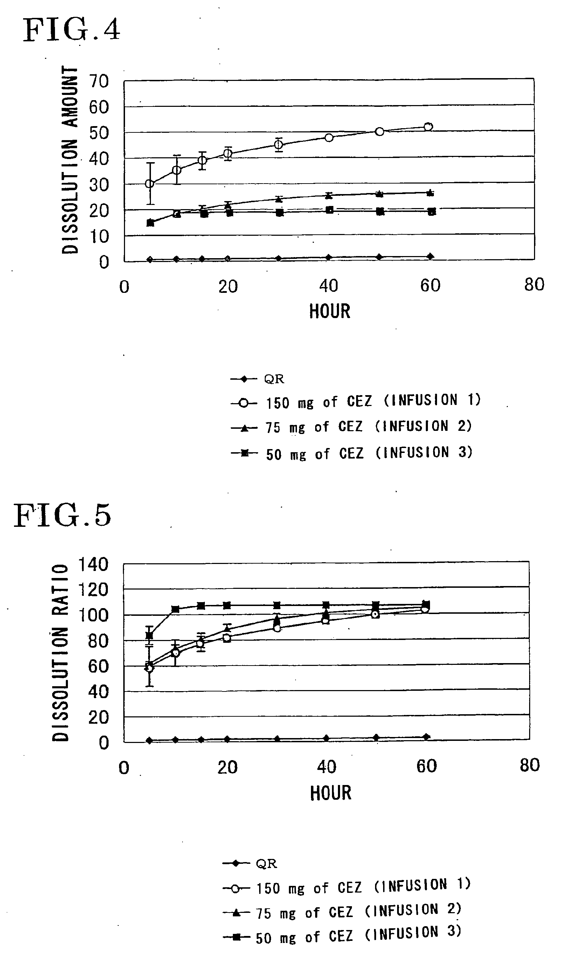

Shown in Figs. 4 and 5 are dissolution amounts and ratios of the above-mentioned infusions 1, 2 and 3 and cefamezin QR as control drug, respectively. As shown in Fig. 5, infusion 3 as one third prescription of CEZ with MCM was instantly started to be dissolved and dissolved out substantially by 100% for 10 minutes. With respect to infusion 1 of CEZ with MCM in an amount same as that of the conventional drug and infusion 2 as one half prescription, the dissolution at substantially 100% was completed for about 40 minutes.

It was revealed from the above that prescriptions with MCM are dissolved out at 100% in a short time even if CEZ ratios are variously changed.

### 2. Test on transition of CEZ concentrations in blood and in milk when administered into cows with various CEZ ratios:

Concentrations in blood and in milk were respectively measured in the same way as that mentioned in the above.

Test drugs shown in Table 6 below, i.e., infusion 2 (one half prescription with 75 mg/3g of CEZ), infusion 3 (one third prescription with 50 mg/3g of CEZ) and cefamezin QR as control drug with 150 mg/3g of CEZ were injected to mammas of six Holstein dairy cows after milking in the morning; this was repeated for 3 days and transition in concentrations in blood and in milk was examined.

**Table 6**

| Test animals | six Holstein dairy cows |
|---|---|
| Test drug | infusion 2: with 2.5% by weight of MCM and 75 mg of CEZ infusion 3: with 2.5% by weight of MCM and 50 mg of CEZ control drug: cefamezin QR (150 mg of CEZ) |
| Administration | Administration through infusion to 4 mammas with each specimen drug (150 mg potency) per mamma; this was repeated for three days |
| Sampling | blood: before administration and 0.5, 1, 2, 3, 4, 6, 8 and 24 hours after administration milk: before administration, at milking (morning and night) in the administration period and 6, 24, 30, 48, 54 and 72 hours after the final administration |
| Analysis | blood serum: HPLC method milk: bioassay method |

Table 7 shows transition of concentration in blood. The blood was extracted before the administration and 0.5, 1, 3, 4, 6, 8 and 24 hours after the 1st day administration. The measurable limit (ND) of the concentration in blood was 0.05 µg (potency)/ml or less.

**Table 7 Transition of concentrations in blood (µg (potency)/mL)**

| Group | Unity code | Before admin. 0 | Time lapsed (hour) | | | | | | |
|---|---|---|---|---|---|---|---|---|---|
| | | | 0.5 | 1 | 2 | 4 | 6 | 8 | 24 |
| Infusion 2 with 75 mg of CEZ | A | ND | ND | 0.017 | ND | ND | ND | ND | ND |
| | B | ND | ND | 0.036 | 0.051 | ND | ND | ND | ND |
| | C | ND | ND | 0.015 | ND | ND | ND | ND | ND |
| | D | ND | 0.018 | ND | ND | ND | ND | ND | ND |
| | E | ND | 0.032 | 0.028 | 0.033 | ND | ND | ND | ND |
| | F | ND | 0.020 | 0.022 | 0.022 | 0.018 | ND | ND | ND |
| Infusion 3 with 50 mg of CEZ | A | ND | 0.0200 | 0.0232 | ND | ND | ND | ND | ND |
| | B | ND | 0.0372 | 0.0289 | 0.0164 | ND | ND | ND | ND |
| | C | ND | ND | ND | ND | ND | ND | ND | ND |
| | D | ND | 0.0422 | 0.0407 | ND | ND | ND | ND | ND |
| | E | ND | 0.0291 | 0.0314 | ND | ND | ND | ND | ND |
| | F | ND | 0.0316 | 0.0380 | ND | ND | ND | ND | ND |
| Control drug cefamezin QR | A | ND | 0.0603 | 0.0843 | 0.0410 | 0.0247 | 0.0250 | 0.0208 | ND |
| | B | ND | ND | ND | 0.0211 | 0.0322 | 0.0228 | 0.0188 | ND |
| | C | ND | ND | ND | 0.0167 | ND | ND | ND | ND |
| | D | ND | 0.0442 | 0.0596 | 0.0349 | 0.0318 | ND | ND | ND |
| | E | ND | 0.0154 | 0.0226 | 0.0433 | 0.0349 | 0.0198 | 0.0203 | ND |
| | F | ND | ND | 0.0167 | 0.0282 | 0.0319 | 0.0182 | ND | ND |

According to the above Table 7, infusions 2 and 3 with MCM appeared fast and eliminated fast; while cefamezin QR became undetectable after 8 hours, infusions 2 and 3 became undetectable after 4 and 2 hours, respectively. Although infusions 2 and 3 had the main ingredient in small quantity and did not greatly affect on concentrations in blood, they exhibited higher concentrations in blood from the viewpoint of their concentrations of main ingredient as low as one half or one third.

Thus, according to infusion with MCM, the main ingredient was effectively utilized without waste so that obtained was the possibility of reducing consumed amount of the main ingredient.

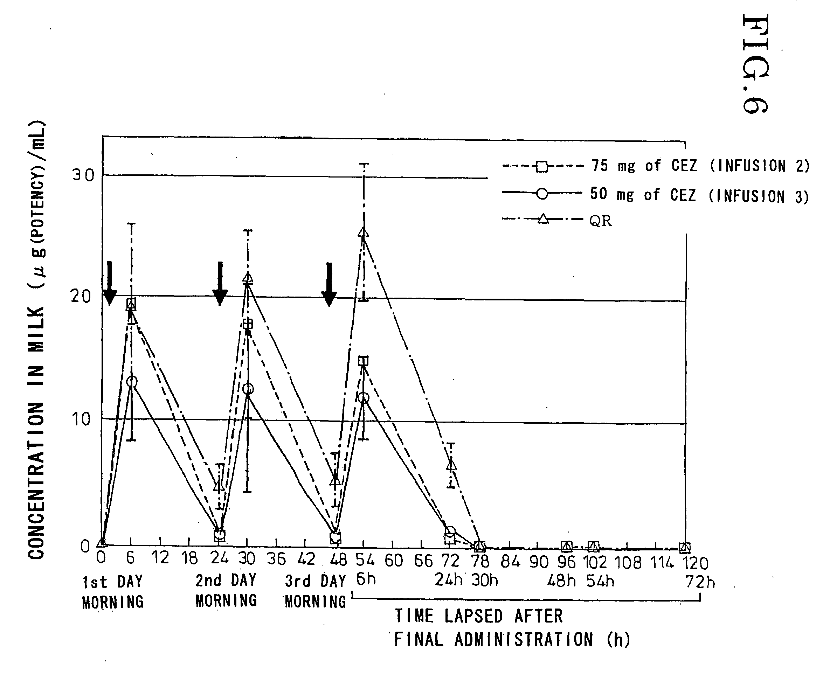

Just like the above-mentioned measurement of transition of the concentrations in blood, transition of concentrations in milk on samples obtained through milking was measured and measurement results are shown in Table 8 and Fig. 6. Milk was got upon milking in the morning and at night during three day's administrations and up to 72 hours after the final administration. Such 72 hours after the final administration was the 3rd day's morning after the final administration. Measurable limit (ND; not detectable) of concentration in milk was 0.05 µg (potency)/ml or less.

**Table 8**

| Transition of concentrations in milk (µg (potency)/mL) | | | | | | | | | | | | |
|---|---|---|---|---|---|---|---|---|---|---|---|---|
| Group | Unity code | 1st day | | 2nd day | | 3rd day | After the final administration | | | | | |
| | | morning | night | morning | night | morning | 6h | 24h | 30h | 48h | 54h | 72h |
| Infusion 2 with 75 mg of CEZ | A | ND | 20.33 | 1.59 | 16.33 | 0.57 | 15.81 | 0.67 | 0.12 | ND | ND | ND |
| | B | ND | 21.97 | 1.84 | 15.66 | 1.14 | 10.24 | 0.92 | 0.29 | ND | ND | ND |
| | C | ND | 26.24 | 3.16 | 28.07 | 2.05 | 18.93 | 1.28 | 0.15 | ND | ND | ND |
| | D | | ND 15.76 | 1.30 | 18.38 | | 1.12 10.79 | 1.56 | 0.47 | ND | ND | ND |
| | E | | ND 10.95 | 0.51 | 9.06 | 0.37 | 16.48 | 0.53 | 0.11 | ND | ND | ND |
| | F | ND | 19.79 | 0.80 | 16.32 | 1.48 | 14.88 | 0.79 | 0.16 | ND | ND | ND |
| | average ±SD | - | 19.17 | 1.53 | 17.30 | 1.12 | 14.52 | 0.96 | 0.22 | - | - | - |
| | | - | 5.27 | 0.93 | 6.16 | 0.61 | 3.39 | 0.39 | 0.14 | - | - | - |
| Infusion 3 with 50 mg of CEZ | A | ND | 16.9630 | 0.3940 | 6.8600 | 0.5450 | 9.9220 | 0.5270 | 0.0570 | ND | ND | ND |
| | B | ND | 12.5500 | 0.7210 | 5.1480 | 0.5430 | 16.6510 | 0.8840 | 0.1130 | ND | ND | ND |
| | C | ND | 9.7650 | 1.3810 | 8.6350 | 0.7410 | 12.0680 | 0.6250 | 0.1740 | ND | ND | ND |
| | D | ND | 14.7070 | 1.1740 | 27.2310 | 1.1190 | 12.4870 | 1.3240 | 0.1890 | ND | ND | ND |
| | E | ND | 18.0560 | 0.5940 | 17.7300 | 0.4470 | 12.6820 | 1.1430 | 0.0600 | ND | ND | ND |
| | F | ND | 5.7410 | 0.9070 | 8.2950 | 1.0930 | 6.8660 | 1.2610 | 0.2280 | ND | ND | ND |
| | average ±SD | - | 12.9637 | 0.8618 | 12.3165 | 0.7480 | 11.7793 | 0.9607 | 0.1368 | - | - | - |
| | | - | 4.6376 | 0.3686 | 8.5163 | 0.2934 | 3.2472 | 0.3353 | 0.0711 | - | - | - |
| Control drug cefamezin QR | A | ND | 23.8740 | 2.9050 | 22.7920 | 3.8280 | 19.5050 | 5.6450 | ND | ND | ND | ND |
| | B | ND | 10.4510 | 5.9370 | 17.7940 | 5.7720 | 30.3120 | 6.6140 | 1.9650 | 0.3510 | 0.0510 | ND |
| | C | ND | 20.1640 | 6.9870 | 26.1430 | 8.9550 | 23.7410 | 9.6260 | 4.4310 | 0.7800 | 0.2880 | ND |
| | D | ND | 27.5280 | 5.1260 | 17.7300 | 4.3210 | 33.3340 | 6.8420 | 0.8830 | 0.0730 | ND | ND |
| | E | ND | 11.7240 | 5.6840 | 19.3590 | 5.7160 | 25.4810 | 5.4150 | 1.4100 | 0.3880 | 0.1110 | ND |
| | F | ND | 21.6560 | 2.6460 | 25.5950 | 3.2080 | 19.7010 | 5.0850 | 0.9770 | 0.1100 | ND | ND |
| | average ±SD | - | 19.2328 | 4.7142 | 21.5688 | 5.3000 | 25.3457 | 6.5378 | - | - | - | - |
| | | - | 6.7911 | 1.8115 | 3.8078 | 2.0619 | 5.6044 | 1.6615 | - | - | - | - |

According to Fig. 6 and Table 8, in any administration group, concentration in milk is the highest upon first milking after each administration and that in the next morning is lower. However, as to concentration at the first milking after each administration, infusions 2 and 3 with MCM exhibited substantially same values in any of 1st, 2nd and 3rd days whereas cefamezin QR exhibited tendency of increase day by day and had the similar tendency of increase in the sample 24 hours after the final administration. Thus, it is considered that cefamezin QR is gradually accumulated in a teat cistern while infusions 2 and 3 has less accumulativeness.

As to concentration in milk, that of cefamezin QR was the highest; that of infusion 2 with one half prescription was the second highest; and that of infusion 3 with one third prescription was the lowest. Since infusion 2, which has main ingredient in an amount half as much as that of cefamezin QR, actually has concentration in milk as much as that of the latter, it was confirmed that prescription with MCM has high dissolubility in comparison with cefamezin QR.

Next, reference is made to drug holiday. As shown in transition of concentrations in milk of Table 8, the final detection of infusions 2 and 3 with MCM was at 30 hours after the final administration whereas cefamezin QR had last detection after 54 hours; that is, the final detection of infusions 2 and 3 is shorter than that of cefamezin QR by 24 hours. Thus, it was indicated that, in comparison with cefamezin QR, infusion with MCM may shorten its drug holiday by 1 day (24 hours).

### 3. Test on residuality of CEZ when main ingredient is of the same amount as that of current prevailing prescription:

As revealed in the above tests, infusions with MCM had tendency of lowering CEZ concentration in milk by repeated administrations whereas no such decline tendency was admitted in administrations of cefamezin QR; thus, it was conjectured that lowering in CEZ concentration in milk is a phenomenon specific to prescription with MCM. Since MCM has absorption acceleration effect and was considered to affect on absorbency of CEZ through repeated administrations, it was thought out that drug holiday may be shortened in ordinary dose regimen and dosage even if the main ingredient is of the same amount as that in current prevailing drugs; thus, the following tests were conducted.

Sunsoft 707 (product of Taiyo Kagaku Co., Ltd.) which is monoester of caprylic acid with 8 carbon atoms was used as MCM to prepare infusion 4 (3g) with composition shown in Table 9.

### [Example 4]

Test drugs used were infusion 1 (with 2.5% by weight of MCM and 150 mg of CEZ) same as that in Example 1 and cefamezin QR (cefazolin oily infusion with no MCM and with 150 mg of CEZ: product of Nippon Zenyaku Kogyo Co., Ltd.) as control drug. Under the conditions shown in Table 9, the respective test drugs were infused to mammas of four Holstein milk cows once a day and for three days to examine transition of CEZ concentrations in milk and blood. Blood was obtained at 0.5, 1, 2, 4, 8 and 12 hours after each administration of 1st to 3rd days and milk was obtained in the morning and at night of 1st to 3rd days and 12, 24, 36, 48, 60, 72 hours after the final administration and measurements were made for the respective materials.

**Table 9**

| Test animal | four Holstein dairy cows |
|---|---|
| test drugs | infusion 1: with 2.5% by weight of MCM and 150 mg of CEZ control drug: cefamezin QR (150 mg of CEZ) |
| Administration | Administration through infusion to 4 mammas with each specimen drug per mamma; this was repeated for three days. |
| Sampling | blood: 0.5, 1, 2, 4, 8 and 12 hours after each administration of 1st through 3rd days milk: at each milking (morning and night) of 1st through 3rd days and 12, 24, 36, 48, 60 and 72 hours after the final administration |
| Analysis | blood serum: HPLC method milk: bioassay method |

### (1) Transition of concentration in milk after administrations of test drugs

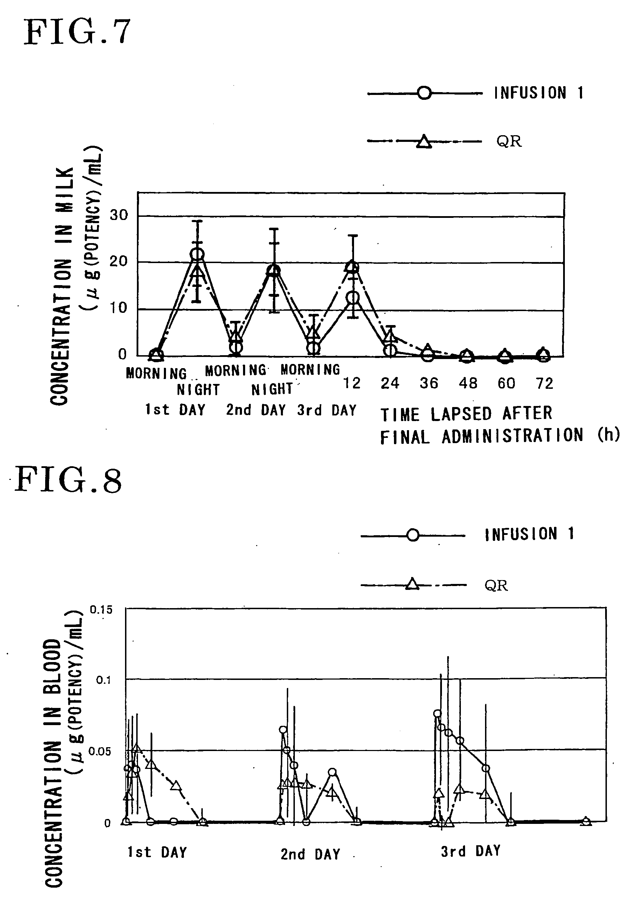

Transition of CEZ concentrations in milk is shown in Table 10 and Fig. 7. Measurable limit (ND; not detectable) of concentration in milk in Table 10 was 0.05 µg (potency)/ml or less.

**Table 10**

| Transition of concentrations in milk (pg (potency)/mL) | | | | | | | | | | | | |
|---|---|---|---|---|---|---|---|---|---|---|---|---|
| Test drug | Unity Code | 1st day | | 2nd day | | 3rd day | After the final administration | | | | | |
| | | morning | night | morning | night | morning | 12 | 24 | 36 | 48 | 60 | 72 |
| Infusion 1 | A | ND | 30.28 | 3.93 | 24.55 | 2.23 | 16.56 | 1.57 | 0.14 | ND | ND | ND |
| | B | ND | 24.55 | 1.47 | 18.77 | 1.21 | 9.76 | 0.53 | 0.06 | ND | ND | ND |
| | C | ND | 18.79 | 1.93 | 19.47 | 1.13 | 15.08 | 1.16 | 0.11 | ND | ND | ND |
| | D | ND | 13.92 | 0.79 | 10.95 | 0.64 | 7.73 | 0.85 | ND | ND | ND | ND |
| | average | - | 21.89 | 2.03 | 18.44 | 1.30 | 12.28 | 1.28 | - | - | - | - |
| | ±SD | - | 7.09 | 1.35 | 5.62 | 0.67 | 4.21 | 0.44 | - | - | - | - |
| Control drug QR | A | ND | 21.18 | 8.34 | 24.08 | 10.12 | 25.85 | 6.58 | 2.35 | 0.36 | 0.11 | ND |
| | B | ND | 23.87 | 3.12 | 26.91 | 4.56 | 23.21 | 4.69 | 0.63 | 0.24 | ND | ND |
| | C | ND | 17.82 | 3.38 | 14.02 | 3.10 | 16.89 | 3.29 | 0.73 | 0.06 | ND | ND |
| | D | ND | 8.94 | 0.62 | 7.71 | 1.17 | 9.95 | 0.93 | 0.11 | ND | ND | ND |
| | average | - | 17.95 | 3.87 | 18.21 | 4.74 | 18.98 | 3.87 | 0.96 | - | - | - |
| | ±SD | - | 6.50 | 3.23 | 8.93 | 3.85 | 7.10 | 2.38 | 0.97 | - | - | - |

### a) Infusion 1 :

In Table 10, upon first milking (at night) at 12 hours after each administration (in the morning) of test drugs in 1st to 3rd days, respective CEZ concentrations in milk (average) were 21.89±7.09 µg (potency)/mL at the first time, 18.44±5.62 µg (potency)/mL at the second time and 12.28±4.21 µg(potency)/mL at the third time; also in the tests, the tendency of CEZ concentration lowering, administration by administration, was confirmed. Moreover, upon second (morning) milking at 24 hours after each administration of test drugs, CEZ concentrations in milk were 2.03±1.35 µg (potency)/mL at the first time, 1.30± 0.67 µg (potency)/mL at the second time and 1.28±0.44 µg (potency)/mL at the third time, which are substantial lowering in comparison with the above-mentioned first milking after each administration.

Final detection of CEZ in milk was at 36 hours after the final administration in one of the four cows; at 48 hours after the final administration, CEZ concentrations for all the cows were lower than measurable limit.

### b) Control drug (cefamezin QR):

In Table 10, upon first milking (at night) at 12 hours after each administration of test drugs in each morning in 1st to 3rd days, respective CEZ concentrations in milk were 17.95±6.50µg (potency)/mL at the first time, 18.21±8.93 µg (potency)/mL at the second time and 18.98± 7.10 µg (potency)/mL at the third time. Moreover, upon second (morning) milking at 24 hours after each administration of test drugs, CEZ concentrations in milk were 3.87±3.23 µg (potency)/mL at the first time, 4.74± 3.85 µg (potency)/mL at the second time and 3.87±2.38 µg (potency)/mL at the third time, which are substantial lowering in comparison with the above-mentioned first milking after each administration.

Final detection of CEZ in milk was at 36 hours after the final administration in one of the four cows, at 48 hours after the final administration in two of the four cows and at 60 hours after the final administration in the remaining cow; thus, at 72 hours after the final administration, CEZ concentrations for all of the cows were lower than measurable limit.

### (2) Transition of concentrations in blood after each administration of test drugs:

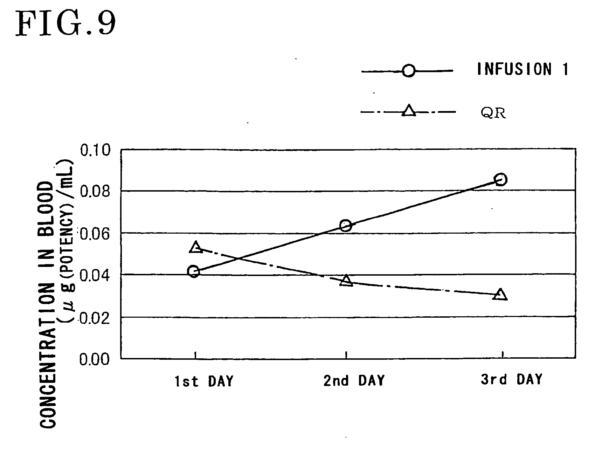

Transition of CEZ concentrations in blood are shown in Fig. 8. Transition of Cmax for respective administrations are shown in Fig. 9.

### a) Infusion 1:

Maximum concentration in blood reached at 0.5-2 hours after each administration of infusion 1; at 24 hours after each administration, all of the cows had concentrations in blood lower than measurable limit. Cmax for the respective administrations were 0.04±0.03 µg (potency)/mL at the first time, 0.06±0.04 µg (potency)/mL at the second time and 0.08±0.04 µg (potency)/mL at the third time.

### b) Control drug (cefamezin QR):

Maximum concentration in blood reached 0.5-8 hours after each administration of contrast drug; at 24 hours after each administration, all of the cows had concentrations in blood lower than measurable limit. Cmax for the respective administrations were 0.05±0.02 µg (potency)/mL at the first time, 0.04±0.01 µg (potency)/mL at the second time and 0.03±0.01 µg (potency)/mL at the third time.

From the above tests and as shown in Table 10, with respect to infusion 1 the final detection in milk was at 36 hours after the final administration and with respect to control drug, at 60 hours after the final administration. Thus, it was indicative just like the above test results that even in infusion 1 which has main ingredient in an amount same as that of current prevailing drugs, there is a possibility of shortening drug holiday by 24 hours in comparison with current prevailing drugs by use of base with MCM.

It was confirmed that lowering of CEZ concentration in milk in repeated administrations noticed only in prescription with MCM was similarly exhibited in the above test. With respect to concentration in blood, Cmax for infusion 1 has tendency of increase, administration by administration; it was considered that absorbency is increased as administrations are repeated. Since concentration in blood itself is low, it is hardly considered that all of lowering in concentration in milk is transferred to increase in concentration in blood; however, it can be envisaged that such increase in absorbency contributes to lowering of concentration in milk.

In addition to the above-mentioned Examples 1-4, Examples 5-9 are shown in Table 11 with different main ingredients and different added amounts of MCM. It was revealed that dissolubility of main ingredient in infusion for mastitis is significantly enhanced by addition of medium-chain fatty acid monoglyceride (MCM) even in an amount as little as 0.5% by weight as shown in example 9. Thus, it was revealed that addition of 0.5% by weight or more of MCM to infusion for mastitis enhances dissolubility of main ingredient, which enhances absorbency, so that its main ingredient can be effectively utilized to reduce consumed amount of main ingredient in comparison with conventional infusion for mastitis.

**Table 11**

| | main ingredient | MCM | additive | oily base | Physical yes properties |
|---|---|---|---|---|---|
| Example 1 (infusion 1) | 150 mg (potency) of cefazolin (CEZ) | 75 mg (2.5% by weight) | 25 mg of food blue No. 1 | canola oil. remainder | Suitable |
| Example 2 (infusion 2) | 75 mg (potency) of cefazolin (CEZ) | 75 mg (2.51 by weight) | 25 mg of food blue No. 1 | canola oil, remainder | Suitable |
| Example 3 (infusion 3) | 50 mg (potency) of cefazolin (CEZ) | 75 mg (2.5% by weight) | 25 mg of food blue No. 1 | canola oil. remainder | Suitable |
| Example 4 (infusion 1) | 150 mg (potency) of cefazolin(CEZ) | 75 mg (2.5% by weight) | 25 mg of food blue No. 1 | canola oil. remainder | Suitable |
| Example 5 | 300 mg (potency) of 300 mg (potency) of tiamulin base | 1500 mg (50% by weight) | 25 mg of food blue No. 1 | canola oil, remainder | Suitable |
| Example 6 | 250 mg (potency) of tiamuline fumarate tiamuline fumarate | 30mg (1.0% by weight) | 25mg of food blue No. 1 | canola oil, remainder | Suitable |
| Example 7 | 300.000 units benzylpenicillin procaine and 300 mg (potency) of kanamycin sulfate | 75 mg (2.5% by weight) | 25 mg of food blue No. 1 | canola oil, remainder | Suitable |
| Example 8 | 300,000 units benzylpenicillin procaine and 300 mg (potency) of sodium dihydrostreptomycine | 75 mg (2.5% by weight) | 25 mg of food blue No. 1 | canola oil, remainder | Suitable |
| Example 9 | 200 mg (potency) of cloxacillin sodium cloxacillin sodium and 75 mg (potency) of ampicillin | 15 mg (0.5% by weight) | 25 mg of food blue No. 1 | canola oil. remainder | Suitable |

### [Example (reference example)

Infusion (3 g) of the invention was prepared by 300 mg (potency) of tiamulin base, 1500 mg (50% by weight) of MCM, 25 mg of food blue No. 1 and canola oil, remainder. Infusion of Example 5 exhibited physical properties suitable as infusion for mastitis.

### [Example (reference example)

Infusion (3 g) of the invention was prepared by 250 mg of tiamuline fumarate, 30 mg (1.0% by weight) of MCM, 25 mg of food blue No. 1 and canola oil, remainder. Infusion of Example 6 exhibited physical properties suitable as infusion for mastitis.

### [Example 7] (reference example)

Infusion (3 g) of the invention was prepared by 300,000 units benzylpenicillin procaine and 300 mg potency of kanamycin sulfate, 75 mg (2.5% by weight) of MCM, 25 mg of food blue No. 1 and canola oil, remainder. Infusion of Example 7 exhibited physical properties suitable as infusion for mastitis.

### [Example (reference example)

Infusion (3 g) of the invention was prepared by 300,000 units benzylpenicillin procaine and 300 mg (potency) of sodium dihydrostreptomycine, 75 mg (2.5% by weight) of MCM, 25 mg of food blue No. 1 and canola oil, remainder. Infusion of Example 8 exhibited physical properties suitable as infusion for mastitis.

### [Example 9] (reference example)

Infusion (3 g) of the invention was prepared from 200 mg (potency) of cloxacillin sodium and 75 mg of (potency) ampicillin, 15 mg (0.5% by weight) of MCM, 25 mg of food blue No. 1 and canola oil, remainder. Infusion of Example 9 exhibited physical properties suitable as infusion for mastitis.

It is to be understood that infusion for mastitis according to the invention is not limited to the above examples and that various changes and modifications may be made without leaving the scope of the invention.

### Industrial Applicability

Infusion for mastitis according to the invention can improve diffusivity or dispersivity of main ingredient, enhance dissolubility of main ingredient to enhance absorbency thereof, so that, in comparison with conventional infusions for mastitis, it has immediate effectivity, can reduce residual period and can reduce consumed amount of main ingredient.

## Claims

1. Infusion for treating mastitis comprising cephem antibiotic as a main ingredient, medium-chain fatty acid monoglyceride having carbon atoms in the range of C8 through C12 and an oily base.

2. Infusion for treating mastitis as claimed in claim 1, wherein an amount of the medium-chain fatty acid monoglyceride is 0.5% by weight or more of total.

## Patentansprüche

1. Infusion zur Behandlung von Mastitis, umfassend ein Cephem-Antibiotikum als Hauptbestandteil, ein Fettsäuremonoglycerid mit mittlerer Kettenlänge mit Kohlenstoffatomen im Bereich von C8 bis C12 und eine ölige Grundlage.

2. Infusion zur Behandlung von Mastitis nach Anspruch 1, wobei der Anteil des Fettsäuremonoglycerids mit mittlerer Kettenlänge 0,5 Gew.-% oder mehr der Gesamtmenge beträgt.

## Revendications

1. Perfusion pour le traitement de mastites comprenant du céphème antibiotique comme ingrédient principal, un monoglycéride d'acide gras à chaîne moyenne ayant un nombre d'atomes de carbone dans la plage allant de C8 à C12.

2. Perfusion pour le traitement de mastites selon la revendication 1, dans laquelle la quantité de monoglycéride d'acide gras à chaîne moyenne est de 0,5 % en poids ou plus par rapport au total.
